# FASCICULE DE BREVET EUROPEEN

(11) **EP 3 976 157 B1**
(45) Date de publication et mention de la délivrance du brevet: **07.06.2023**
(21) Numéro de dépôt: 20739744.9
(22) Date de dépôt: 22.05.2020
(51) Int. Cl.: A61M 25/09, A61M 25/00, A61B 50/30, A61B 50/33

(54) **DISPOSITIF DE STOCKAGE POUR FIL-GUIDE**
SPEICHERVORRICHTUNG FÜR EINEN FÜHRUNGSDRAHT
STORAGE DEVICE FOR A GUIDE WIRE

(30) Priorité: 24.05.2019 FR 1905538
(43) Date de publication de la demande: 06.04.2022
(73) Titulaire: Chatel, Didier, 37390 Mettray (FR)
(72) Inventeur: Chatel, Didier, 37390 Mettray (FR)
(74) Mandataire: Santarelli
(86) Numéro de dépôt international: PCT/FR2020/050856
(87) Numéro de publication internationale: WO 2020/240123

(56) Documents cités:
- WO-A1-02/087996
- US-A1- 2016 206 394
- US-A1- 2016 354 576

## Description

La présente invention concerne les dispositifs de rangement peropératoire de guides utilisés lors de procédures interventionnelles endovasculaires ou endocardiaques.

Lors de ces interventions, il est d'usage d'utiliser des guides ou filsguides appelés « guide wires » en anglais. Ces guides sont des « filins » pouvant être réalisés en matière métallique ou pas, et ayant une flexibilité variable.

Dans la pratique, les guides sont conditionnés stérilement et délivrés dans des étuis. Sortis des étuis, les guides sont introduits dans les vaisseaux de patients pour servir de guides de sondes utiles à la réalisation d'imagerie médicale ou à des actes thérapeutiques. A titre d'exemples, les guides peuvent être utilisés pour des interventions ou procédures endovasculaires telles que : l'implantation d'une valve aortique par voie percutanée (TAVI), la cardiologie interventionnelle coronaire, les endoprothèses pour le traitement d'anévrismes de l'aorte, etc.

En général, une fois retirés des vaisseaux des patients, les guides sont enroulés sur eux-mêmes et déposés en vrac dans des bassines contenant du sérum physiologique. Cependant, cette manière de ranger les guides n'est pas optimale dans le mesure où la reprise des guides pour une seconde phase de la procédure interventionnelle impose de les chercher dans le « vrac » de la bassine.

Dans ce contexte, on connait des dispositifs de rangement des guides permettant de stocker les guides dans l'attente d'une réutilisation ultérieure. Le document US 2016 020 6394 divulgue un dispositif comportant une cuve et un cadre. Le cadre comporte plusieurs compartiments, chaque compartiment étant adapté à recevoir un guide. Chaque guide doit être enroulé avant d'être rangé dans un compartiment. Ainsi, le rangement des guides requiert l'utilisation de deux mains.

La présente invention a pour but de résoudre au moins l'un des inconvénients précités. Le dispositif de stockage selon l'invention est défini dans la revendication 1. Des modes de réalisation sont définis dans les revendications dépendantes.

L'invention concerne un dispositif de stockage pour fil-guide, comportant une cuve et au moins un cadre amovible adapté à être disposé dans la cuve, ladite cuve étant destinée à contenir du sérum physiologique. Le cadre comporte deux parois opposées formant entre elles un volume interne de stockage et comprend un orifice d'introduction formé dans le volume interne et s'étendant depuis une première extrémité des parois en direction d'une deuxième extrémité des parois opposée à ladite première extrémité.

Selon l'invention, le cadre comprend une ouverture traversant le cadre d'une paroi à l'autre, et une gorge sensiblement annulaire formée dans le volume interne et communiquant avec l'orifice d'introduction. La gorge débouche sur l'ouverture sur une partie de son périmètre, appelée partie ouverte, et est séparée de l'ouverture sur le reste de son périmètre. La cuve comporte des moyens de positionnement adaptés à placer le cadre dans une position de rangement dans laquelle la partie ouverte de la gorge se trouve au moins partiellement à l'intérieur de la cuve et l'orifice d'introduction s'étend en dehors de la cuve. La cuve comporte en outre des moyens d'arrêt adaptés à bloquer la translation du cadre dans des directions d'introduction et d'extraction du fil-guide dans le cadre lorsque le cadre est dans la position de rangement.

Le dispositif ainsi constitué permet l'introduction et l'extraction du guide du cadre avec une seule main puisque le cadre est bloqué en translation dans les directions d'introduction et d'extraction du fil-guide grâce aux moyens d'arrêt. Le dispositif ainsi constitué est suffisamment stable en position de rangement pour permettre à un opérateur de ne pas avoir à tenir le cadre pendant l'introduction et l'extraction du fil-guide dans le cadre.

Par ailleurs, le guide peut être facilement introduit via l'orifice d'introduction et poussé dans la gorge.

La forme annulaire de la gorge facilite le rangement et l'enroulement du guide à l'intérieur du cadre. Le guide suit les parois de la gorge de sorte à être correctement rangé et enroulé dans le volume interne du cadre.

Le rangement d'un seul guide par cadre permet par ailleurs de retrouver facilement chaque guide. De plus, en fin de procédure, le guide peut être définitivement conditionné et rangé dans son cadre correspondant afin d'être jeté. En d'autres termes, chaque cadre et chaque guide sont de préférence à usage uniques, ce qui permet donc un conditionnement propre et ordonné pour la mise en déchet.

En outre, le dispositif permet de ranger le guide tout en le trempant dans le sérum physiologique. En effet, la gorge débouchant sur l'ouverture sur une partie de son périmètre, lorsque le guide passe par la partie ouverte pendant le rangement dans le cadre, ledit guide est trempé dans ledit sérum. Cela permet d'humidifier les guides lors du stockage du guide dans le cadre ainsi que lors de l'extraction du guide du cadre.

Enfin, le cadre est amovible, ce qui permet de le déplacer et le manipuler au besoin. Le guide n'a donc pas besoin d'être complètement retiré du cadre pour commencer à être introduit dans le corps d'un patient. Le dispositif de stockage permet ainsi d'économiser une manipulation de guide et conséquemment de prévenir des fautes de manipulation pouvant être sources de fautes d'asepsie et donc source d'infections ; de telles fautes obligeant à utiliser un nouveau guide. Cela permet donc d'économiser le nombre de guides utilisés par procédures.

Selon une caractéristique, les moyens de positionnement sont adaptés à bloquer la translation du cadre selon une direction latérale.

Cela rend le cadre encore plus stable en position de rangement et facilite donc d'autant plus l'introduction et l'extraction du guide du cadre à une seule main.

Selon une caractéristique, un jeu sépare les moyens de positionnement d'au moins une paroi du cadre lorsque le cadre est dans la position de rangement, le jeu étant configuré de sorte à ce que les parois du cadre butent contre les moyens de positionnement lors de l'extraction et l'introduction du fil-guide dans le cadre.

Le cadre est ainsi bloqué en translation latérale dès lors que les parois butent contre les moyens de positionnement. Le jeu présente ainsi l'avantage de faciliter l'introduction et le positionnement du cadre dans la cuve tout en permettant de maintenir, au moyen de la butée, le cadre suffisamment stable dans la cuve pour que l'introduction et l'extraction du fil-guide du cadre puisse se faire à une seule main.

Selon une caractéristique, les parois du cadre sont en appui contre les moyens de positionnement lorsque le cadre est dans la position de rangement.

En d'autres termes, les moyens de positionnement sont configurés de sorte à ce que lorsque le cadre est en position de rangement, aucun jeu n'existe entre les parois et les moyens de positionnement. Le cadre est ainsi bloqué en translation latérale.

Selon une caractéristique, les moyens de positionnement comprennent un élément d'assemblage adapté à coopérer par complémentarité de formes avec le cadre.

L'élément d'assemblage peut être adapté à bloquer la translation latérale du cadre au moins dans un sens.

Selon une caractéristique, les moyens de positionnement sont adaptés à immobiliser le cadre lorsque le cadre est dans la position de rangement. Par exemple, les moyens de positionnement peuvent être une ou plusieurs pinces adaptées à maintenir en place le cadre.

Le cadre étant complètement immobilisé, un opérateur peut alors introduire ou extraire le fil-guide du cadre sans être gêné par un mouvement du cadre.

Le blocage en translation selon la direction latérale, dans un sens, dans les deux sens ou l'immobilisation complète du cadre permet de rendre le cadre plus stable dans sa position de rangement, ce qui facilite d'autant plus l'introduction et l'extraction du cadre à une seule main.

Selon une autre caractéristique, la partie ouverte de la gorge comprend des ponts espacés le long de ladite partie ouverte et s'étendant d'un côté à un autre côté de la gorge.

Lorsque le guide est introduit dans le cadre, le guide est trempé dans le sérum lorsqu'il se trouve au niveau de la partie de la gorge débouchant sur l'ouverture, c'est-à-dire au niveau des portions de la gorge séparées par les ponts.

Selon une caractéristique, la gorge comprend une partie supérieure formée dans une portion supérieure du cadre et une partie inférieure formée dans une portion inférieure du cadre, les parties supérieure et inférieure de la gorge étant semi-annulaires, la gorge étant fermée sur la partie supérieure et débouchant sur l'ouverture sur une ou plusieurs portions de la partie inférieure, l'orifice d'introduction s'étendant dans la portion supérieure du cadre.

L'orifice s'étendant dans la partie supérieure fermée du cadre, le guide est correctement guidé dans la gorge, évitant ainsi de sortir du cadre. En effet, sous son propre poids, le guide pourrait avoir tendance à sortir du cadre si la partie supérieure était ouverte. La partie inférieure peut être partiellement ou entièrement ouverte puisque le poids du guide le fait tomber vers le fond de la partie inférieure de la gorge.

Selon une caractéristique, l'ouverture est sensiblement circulaire.

L'ouverture suit ainsi la forme sensiblement annulaire de la gorge. Cela permet au guide d'être parfaitement humidifié par le sérum physiologique dans la partie de la gorge débouchant sur l'ouverture.

Selon une caractéristique, les parois du cadre sont courbes.

Ainsi, une plus grande partie du cadre et donc du guide peut être trempée dans le sérum physiologique se trouvant dans la cuve. En outre, cela permet de former des cadres de grandes dimensions et donc de grand diamètre sans pour autant augmenter la hauteur du cadre. La mise en place du guide dans un cadre de grand diamètre est donc encore plus facilité sans que la hauteur du cadre et donc du dispositif de stockage ne soit gênante.

Selon une caractéristique, la cuve comporte au moins deux saillies s'étendant dans un fond de la cuve et configurées pour recevoir entre elles le cadre, les saillies formant les moyens de positionnement, un côté de la cuve s'étendant en regard de la deuxième extrémité des parois du cadre et formant les moyens d'arrêt.

Selon une caractéristique, le dispositif comporte un élément introducteur communiquant avec le cadre à travers l'orifice d'introduction.

L'élément introducteur évite à un utilisateur de devoir chercher l'orifice d'introduction et facilite ainsi l'introduction du guide dans le cadre.

Selon une caractéristique, l'élément introducteur comprend une extrémité de montage complémentaire à l'orifice d'introduction du cadre, l'extrémité de montage étant adaptée à s'emboiter dans l'orifice d'introduction du cadre.

Selon une caractéristique, l'élément introducteur présente une forme sensiblement tronconique.

La forme tronconique étant une forme évasée, le guide est ainsi guidé vers l'orifice d'introduction facilitant ainsi l'introduction du guide dans le cadre. La forme tronconique donne une meilleure visibilité à l'utilisateur vers l'orifice d'introduction.

Selon une caractéristique, l'élément introducteur comporte une portion tronconique et un bec d'introduction prolongeant la portion tronconique au niveau d'une extrémité évasée.

L'élément introducteur ayant une portion en forme de bec, cela facilite le guidage du guide vers l'orifice d'introduction.

Selon une caractéristique, l'élément introducteur et le cadre sont formés d'un seul tenant.

D'autres particularités et avantages de l'invention apparaîtront encore dans la description ci-après en référence aux dessins annexés, donnés à titre d'exemples non limitatifs :
- la figure 1 est une vue en perspective d'un fil-guide ;
- la figure 2 est une vue en perspective d'un dispositif de stockage pour fil-guide selon un premier mode de réalisation ;
- la figure 3 est une vue éclatée de la figure 2 ;
- la figure 4 est une vue en perspective d'un cadre du dispositif de stockage de la figure 2 ;
- la figure 5 est une vue inversée en perspective d'un cadre selon un deuxième mode de réalisation ;
- la figure 6 est une vue en coupe d'un dispositif de stockage ayant un cadre selon un troisième mode de réalisation ;
- la figure 7 est une vue de détail d'un élément introducteur du dispositif de stockage de la figure 2 ; et
- la figure 8 est une vue en perspective d'un élément introducteur selon un autre mode de réalisation.

Dans la suite du document, les termes longitudinal et latéral sont utilisés relativement aux figures et ne doivent donc en aucun cas être compris de manière restrictive. Les directions longitudinale et latérale sont perpendiculaires entre elles.

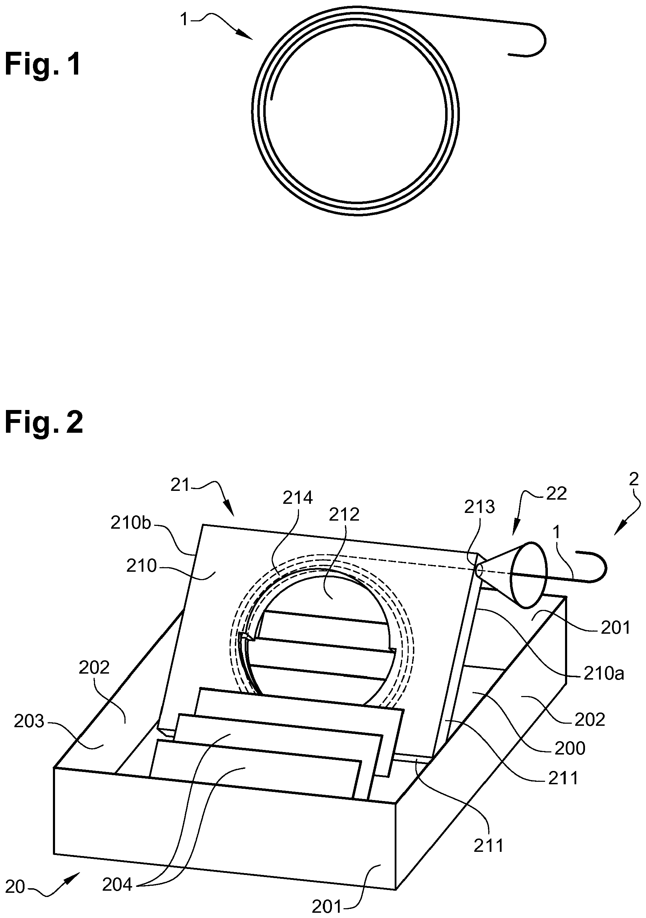

La figure 1 représente un fil-guide également appelé guide 1. Le guide 1 présente une forme de fil.

Le guide 1 est réalisé dans un matériau flexible. La flexibilité du guide 1 permet de l'enrouler facilement comme visible sur la figure dans le but de le stocker par exemple.

Le guide 1 est par exemple métallique ou réalisé en tout autre matériau adéquat.

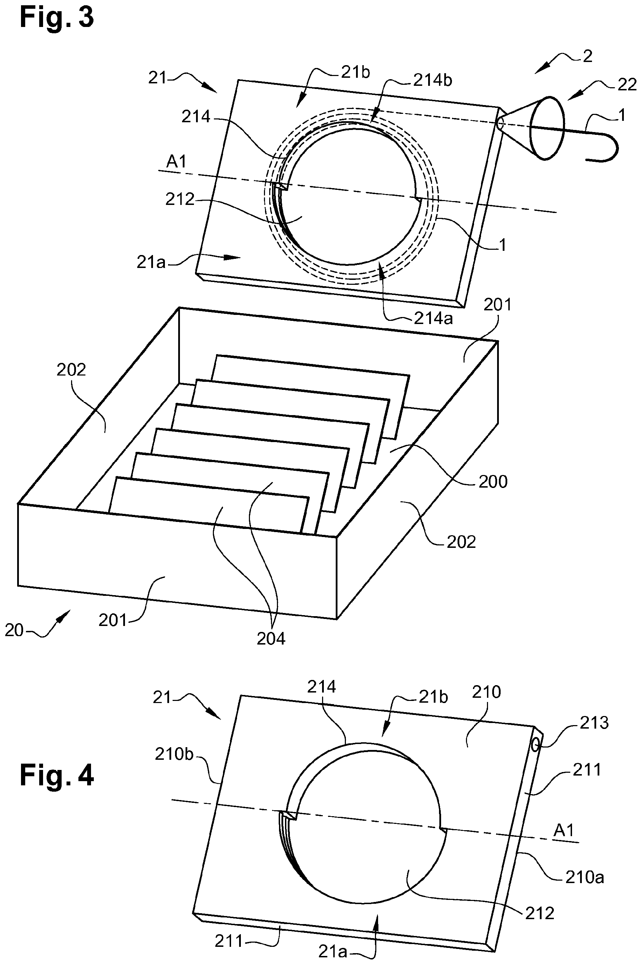

Les figures 2 et 3 représentent en vue de perspective un dispositif de stockage 2 pour guide selon un mode de réalisation. Le dispositif de stockage 2 comporte une cuve 20 et au moins un cadre 21. Dans cet exemple, le dispositif de stockage 2 comporte un seul cadre 21 et comporte en outre un élément introducteur 22.

Bien entendu, le nombre de cadres et d'éléments introducteurs peut varier.

La cuve 20 présente ici une forme de boîte parallélépipédique ouverte sur le dessus. La cuve 20 comporte un fond 200, des côtés longitudinaux 201 et des côtés latéraux 202. Le fond 200 présente une forme rectangulaire. Les côtés longitudinaux 201 et latéraux entourent le fond 200 et s'étendent perpendiculairement au fond 200.

Les côtés longitudinaux 201 et latéraux présentent une hauteur suffisamment grande de sorte à permettre à la cuve 20 d'être remplie de sérum physiologique (non représenté sur ces figures).

Bien entendu, la cuve 20 peut présenter d'autres formes. La cuve 20 peut présenter par exemple des contours carrés ou arrondis.

La cuve 20 comporte des moyens d'arrêt 203 et moyens de positionnement 204. Les moyens de positionnement 204 sont adaptés à placer le cadre dans une position de rangement. Les moyens d'arrêt 203 sont adaptés à bloquer la translation du cadre 21 dans des directions d'introduction et d'extraction du fil-guide dans le cadre 21 lorsque le cadre 21 est dans la position de rangement.

On entend par directions d'introduction et d'extraction du guide, les directions dans lesquels se déplace le cadre lors de l'introduction et l'extraction du guide. Dans l'exemple décrit, les directions d'introduction et d'extraction du guide se confondent avec la direction longitudinale.

Dans cet exemple, la cuve 20 comporte des saillies 204. Les saillies 204 s'étendent dans le fond 200 de la cuve 20. Les saillies 204 constituent ici les moyens de positionnement 204. Les saillies 204 sont ici au nombre de six.

Les saillies 204 présentent ici la forme de plaques rectangulaires. Les saillies 204 s'étendent parallèlement entre elles. Un écart ou espace est formé entre chaque saillie. L'écart entre chaque saillie est au moins égal à l'épaisseur du cadre 21. L'écart est ici le même entre toutes les saillies 204. L'écart est dimensionné de sorte à permettre de recevoir le cadre 21 entre deux saillies 204 tout en empêchant le cadre 21 de translater selon la direction latérale. En d'autres termes, lorsque le cadre 21 est disposé dans la cuve 20, le cadre 21 est en appui contre deux saillies 204. Les dimensions des saillies 204 et en particulier dans cet exemple de réalisation, la longueur des saillies 204 est également choisie de sorte à empêcher la translation du cadre 21 selon la direction latérale.

Bien entendu, le nombre, la forme, la disposition et les dimensions des saillies 204 peuvent varier. A titre d'exemple non limitatif, un jeu peut exister entre le cadre 21 et les saillies 204 lorsque le cadre 21 est dans la position de rangement. Les moyens de positionnement 204 peuvent comprendre un élément d'assemblage adapté à coopérer par complémentarité de formes avec le cadre 21.

Dans le mode de réalisation illustré, lorsque le cadre 21 est disposé dans la cuve 20 dans la position de rangement, le cadre 21 est en butée contre les deux côtés latéraux 202. Les deux côtés latéraux 202 forment ainsi les moyens d'arrêt 203. Le cadre 21 est alors bloqué dans les deux sens de translation longitudinale vers les deux côtés latéraux 202.

Dans cet exemple de réalisation précis, la direction longitudinale correspond à la direction d'introduction et d'extraction du guide du cadre.

Bien entendu, le moyen d'arrêt peut être de tout autre type, forme et nombre. Dans un autre exemple de réalisation,

Le cadre 21 comporte des parois 210 formant entre elles un volume interne de stockage dans lequel est rangé le guide 1. Le cadre 21 comporte ici deux parois 210 et des bords périphériques 211. Les parois 210 comportent chacune une première extrémité 210a et une deuxième extrémité 210b opposée à la première extrémité 21 0a. Les bords périphériques 211 entourent les parois 210. Les parois 210 sont sensiblement parallèles entre elles et les bords périphériques 211 sont deux à deux sensiblement parallèles entre elles de sorte à ce que le cadre 21 présente une forme sensiblement parallélépipédique.

Le cadre 21 peut être fictivement divisé en deux portions ; une portion inférieure 21a et une portion supérieure 21b symétriquement séparées par un axe médian A1 s'étendant parallèlement à la direction longitudinale.

Le cadre 21 comprend une ouverture 212 visible aux figures 2, 3 et 4. L'ouverture 212 est ici à contour sensiblement circulaire. L'ouverture 212 est ici formée dans les parois 210. L'ouverture 212 est traversante, c'est-à-dire que l'ouverture 212 traverse les parois 210.

Le cadre 21 comprend également un orifice d'introduction 213 bien visible à la figure 4. L'orifice d'introduction 213 s'étend depuis la première extrémité 210a vers la deuxième extrémité 210b des parois 210. L'orifice d'introduction 213 est formé dans l'un des bords périphériques 211. L'orifice d'introduction 213 est formé dans la portion supérieure 21b du cadre 2. L'orifice d'introduction 213 est destiné à recevoir le guide 1.

Le cadre 21 comprend en outre une gorge 214. La gorge 214 est formée dans le volume interne du cadre 21 et est adjacente à l'ouverture 212. La gorge 214 est sensiblement annulaire et présente sensiblement un même diamètre qu'un diamètre de l'ouverture 212. La gorge 214 communique avec l'orifice d'introduction 213. La gorge débouche sur l'ouverture 212 sur une partie du périmètre de l'ouverture 212, appelée partie ouverte. En d'autres termes, la gorge 214 est reliée à l'orifice d'introduction 213 et est partiellement ouverte.

La gorge 214 comporte une partie inférieure 214a et une partie supérieure 214b séparées par l'axe médian A1. Dans le mode de réalisation des figures 2, 3 et 4, la gorge 214 est complètement fermée sur la partie supérieure 214b. En d'autres termes, la partie supérieure 214b de la gorge 214 ne communique pas avec l'ouverture 212. En outre, la gorge 214 débouche sur l'ouverture 212 sur la partie inférieure 214a.

Bien entendu, la partie supérieure 214b de la gorge 214 peut être fermée uniquement partiellement, par exemple aux deux-tiers.

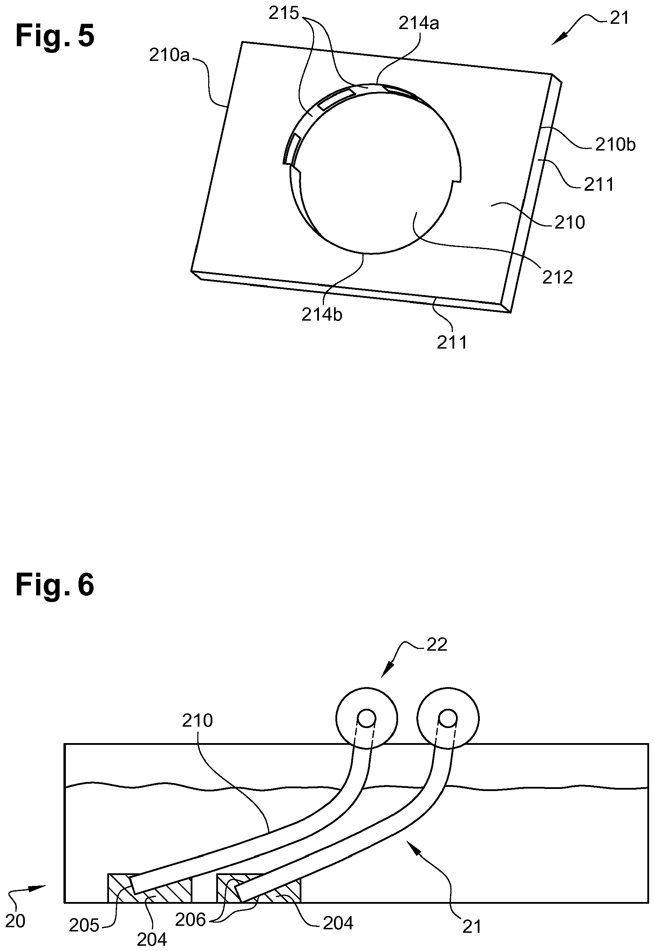

La figure 5 représente un autre mode de réalisation du cadre dans lequel la gorge 214 débouche sur l'ouverture 212 au niveau de plusieurs portions. En particulier, la gorge 214 débouche au niveau de plusieurs portions de la partie inférieure 214a. Une languette ou pont 215 du cadre 21 sépare chaque portion de sorte à fermer localement la gorge 214. En d'autres termes, la partie de la gorge 214 débouchant sur l'ouverture, ou partie ouverte, présente les ponts 215 espacés le long de ladite partie ouverte. Chaque pont 215 s'étend d'un côté à un autre côté de la gorge 214.

La figure 6 représente encore un autre mode de réalisation du cadre dans lequel les parois 210 du cadre 21 sont courbes. Lorsque le cadre 21 est disposé dans la cuve 20 dans la position de rangement, les parois 210 présentent une forme convexe.

Dans cet exemple, la cuve 20 comporte des saillies 204, chaque saillie 204 comprenant une zone de réception 205 s'étendant obliquement de sorte à recevoir le cadre 21. Les zones de réception 205 sont formées de sorte à permettre la disposition par complémentarité de formes d'une partie du cadre 21. Lorsque le cadre 21 est dans la position de rangement, les parois 210 sont en appui contre des faces 206 de la zone de réception 205.

Il est ainsi possible de former des cadres de grandes dimensions et donc dans cet exemple ayant une gorge 214 de grand diamètre facilitant la mise en place des guides dans les cadres, sans pour autant augmenter la hauteur du dispositif. Cela évite d'obtenir un dispositif de stockage ayant une hauteur gênante dans la zone opératoire si elle est trop importante.

Les termes supérieure et inférieure sont utilisés relativement aux figures 2 et 3, et servent uniquement à faciliter la compréhension de l'invention.

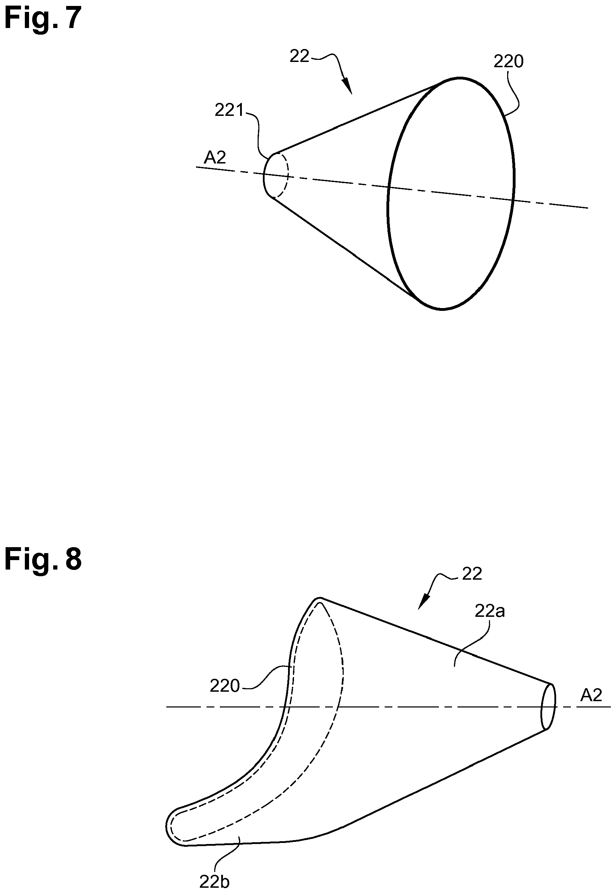

La figure 7 représente en vue de détail l'élément introducteur 22 selon le mode de réalisation de la figure 2. L'élément introducteur 22 est ouvert à ses deux extrémités. L'élément introducteur 22 présente dans l'exemple illustré à cette figure une forme sensiblement tronconique. L'élément introducteur 22 s'étend autour d'un axe de révolution A2, i.e. l'élément introduction est symétrique par rapport à l'axe de révolution A2.

L'élément introducteur 22 comprend une première extrémité 220 et une deuxième extrémité 221 ouvertes. La première extrémité, également appelée extrémité d'introduction 220 ou extrémité évasée, permet à un utilisateur d'introduire le guide 1. La deuxième extrémité, également appelée extrémité de montage 221, est destinée à être montée au cadre 21 et constitue le point d'entrée du guide 1 dans le cadre 21.

L'extrémité de montage 221 de l'élément introducteur 22 est à contour circulaire. L'extrémité de montage 221 présente sensiblement le même diamètre que le diamètre de l'orifice d'introduction 213 du cadre 21. L'extrémité de montage 221 présente ainsi une forme et des dimensions complémentaires à la forme et dimensions de l'orifice d'introduction 213.

La forme évasée de l'élément introducteur 22 facilite l'introduction du guide dans le cadre 21 et permet à l'utilisateur de ne pas avoir à viser l'orifice d'introduction 213 pouvant être de petite dimension.

L'élément introducteur 22 est également représenté à la figure 8 selon un autre mode de réalisation. L'élément introducteur comporte une portion tronconique 22a ayant une extrémité évasée 220 et une extrémité de montage 221, et un bec d'introduction 22b prolongeant ladite portion tronconique 22a au niveau de l'extrémité évasée 220. La portion tronconique 22a s'étend atour de l'axe de révolution A2. Dans cet exemple, l'élément introducteur 22 est asymétrique.

La forme quasi-plane d'une portion du bec d'introduction 22b permet de guider l'introduction du guide 1 dans l'élément introducteur 22 et vers l'orifice d'introduction 213. L'élément introducteur 22 du mode de réalisation de la figure 6 présente une forme suffisamment évasée pour permettre une introduction facile du guide 1 dans le cadre 21.

Dans ces exemples de modes de réalisation, l'élément introducteur 22 est monté au cadre 21 par emboitement. Bien entendu, l'élément introducteur 22 peut être collé ou fixé solidairement au cadre 21. L'élément introducteur 22 peut également être formé d'un seul tenant avec cadre 21.

Dans un exemple de réalisation, le dispositif de stockage 2 et en particulier le cadre 21 peut être réalisé par impression tridimensionnelle. Dans un autre exemple, le dispositif de stockage peut être réalisé par moulage. En particulier, le cadre 21 peut être réalisé par moulage. Dans ce dernier cas, le cadre peut être divisé transversalement en deux parties symétriques formées chacune par moulage et collées entre elles.

Lors du stockage du guide 1 dans le dispositif de stockage 2, le cadre 21 est disposé dans la cuve 20 et positionné grâce aux moyens de positionnement 204. Le cadre 21 est bloqué en translation dans les directions d'introduction et d'extraction du fil-guide grâce aux moyens d'arrêt 203. En fonction des modes de réalisation, le cadre 21 peut être complètement ou partiellement immobilisé dans la cuve 20.

L'élément introducteur 22 est éventuellement monté au cadre 21. L'élément introducteur 22 peut être monté au cadre 21 avant la disposition du cadre 21 dans la cuve 20, après la disposition du cadre 21 dans la cuve 20 ou ne pas être monté du tout.

Le guide 1 est ensuite introduit dans le cadre 21 par un utilisateur. Le guide 1 peut être introduit dans le cadre 21 soit directement au travers de l'orifice d'introduction 213 soit au moyen de l'élément introducteur 22. Le guide 1 peut être introduit à une main puisque les moyens d'arrêt 203 empêchent le cadre 21 de bouger en translation dans les directions d'introduction et d'extraction du fil-guide 1 dans le cadre 21.

En particulier dans le mode de réalisation représenté aux figures 2 et 3, la deuxième extrémité 210b des parois 210 du cadre 21 est en butée contre les moyens d'arrêt 203. En outre, les parois 210 du cadre 21 sont en appui contre les moyens de positionnement 204. Dans un autre exemple de réalisation, le cadre 21 peut être bloqué en translation longitudinale dans les deux sens. Cela facilite d'autant plus l'introduction et le retrait du guide à une seule main puisque le cadre 21 est parfaitement maintenu fixe dans la cuve 20.

En premier lieu, le guide 1 suit la gorge 214 jusqu'à ce qu'une première partie du guide 1 s'enroule sur un tour de la gorge 214. L'utilisateur continue à introduire le guide 1 de sorte à ce que la quasi-totalité du guide 1 soit enroulée dans la gorge 214 et seule une portion reste à l'extérieur du cadre 21 comme cela est bien visible aux figures 2 et 3. Dans ces figures, les parties du guide 1 dans le volume interne du cadre 21 sont représentées en pointillés. La portion du guide 1 restant à l'extérieur du cadre 21 permet à l'utilisateur de sortir le guide 1 du cadre 21 en tirant ladite portion, par exemple pour son utilisation lors d'une procédure interventionnelle.

La gorge 214 étant partiellement fermée, dans les exemples décrits sur toute la partie supérieure 214b, le guide 1 est bien guidé le long de la gorge 214 et ne peut sortir vers l'ouverture 212 pendant son stockage ou rangement dans le cadre.

La cuve 20 est préalablement remplie au moins partiellement de sérum physiologique. Ainsi, lorsque le guide 1 est enroulé dans le cadre 21 ou retirée du cadre 21, la quasi-totalité du guide 1 passe par la partie ou portions débouchant sur l'ouverture 212 de sorte à permettre au guide 1 d'être trempé dans le sérum physiologique pendant la procédure de stockage.

Dans le mode de réalisation illustré à la figure 6, grâce à la forme courbe des parois 210, une plus grande partie du cadre 21 se trouve dans le sérum physiologique du dispositif et donc une plus grande partie du guide stocké dans le cadre est baignée dans le sérum physiologique.

Le cadre 21 est amovible et peut donc être déplacé au besoin dans la cuve 20.

Dans le cas où l'élément introducteur 22 est monté au cadre 21 par emboitement, l'élément introducteur 22 est également amovible du cadre 21, i.e. l'élément introducteur 22 peut être montée au cadre 21 ou ôté selon le besoin. Cela permet d'utiliser un même élément introducteur 22 pour plusieurs cadres 21.

Bien entendu, la présente invention n'est pas limitée aux modes de réalisation décrits et illustrés.

A titre d'exemple, les moyens de positionnement 204 peuvent également être des pinces saillant du fond 200 de la cuve 20.

Le moyen d'arrêt 203 peut être une saillie faisant relief du fond 200 de la cuve 20 et s'étendant selon une direction latérale.

La cuve 20 peut comprendre un côté ou paroi intermédiaire séparant la cuve en une première partie et une deuxième partie. La première partie peut être adaptée à recevoir le cadre 21. Ainsi, la première partie de la cuve peut comprendre les moyens de positionnement et les moyens d'arrêt. Le côté intermédiaire peut constituer un moyen d'arrêt. La deuxième partie peut être adaptée à former un compartiment pour recevoir une solution désinfectante et/ou antiseptique pouvant être utilisée par l'opérateur pour s'humidifier les mains ou y placer d'autres outils.

La présente invention propose ainsi un dispositif de stockage permettant le rangement propre, isolé et conséquemment répertorié de chaque guide, l'obtention d'un champ opératoire clair et ordonné. Le dispositif de stockage permet également d'éviter les fautes d'asepsies sur guides, et donc les changements inopportuns de guides. Le stockage du guide dans son cadre se fait facilement et au moyen d'une seule main. Enfin, le conditionnement définitif des guides rangés dans les cadres à usage unique en fin de procédure permet un conditionnement propre pour la mise en déchet des guides ainsi rangés dans les cadres.

## Revendications

1. Dispositif de stockage pour fil-guide (1), comportant une cuve (20) et au moins un cadre (21) amovible adapté à être disposé dans la cuve (20), ladite cuve (20) étant destinée à contenir du sérum physiologique, ledit cadre (21) comportant deux parois (210) opposées formant entre elles un volume interne de stockage et comprenant un orifice d'introduction (213) formé dans ledit volume interne et s'étendant depuis une première extrémité (210a) desdites parois (210) en direction d'une deuxième extrémité (210b) desdites parois (210) opposée à ladite première extrémité (210a), ledit cadre (21) comprenant une ouverture (212) traversant le cadre (21) d'une paroi (210) à l'autre, et une gorge (214) sensiblement annulaire formée dans ledit volume interne et communiquant avec ledit orifice d'introduction (213), ladite gorge (214) débouchant sur l'ouverture (212) sur une partie de son périmètre, appelée partie ouverte, et étant séparée de l'ouverture (212) sur le reste de son périmètre, et ladite cuve (20) comportant des moyens de positionnement (204) adaptés à placer le cadre (21) dans une position de rangement dans laquelle la partie ouverte de la gorge (214) se trouve au moins partiellement à l'intérieur de la cuve (20) et l'orifice d'introduction (213) s'étend en dehors de la cuve (20), ladite cuve (20) comportant en outre des moyens d'arrêt (203) adaptés à bloquer la translation du cadre (21) dans des directions d'introduction et d'extraction du fil-guide dans le cadre (21) lorsque ledit cadre (21) est dans la position de rangement.

2. Dispositif de stockage selon la revendication 1, dans lequel les moyens de positionnement (204) sont adaptés à bloquer la translation du cadre (21) selon une direction latérale.

3. Dispositif de stockage selon l'une des revendications 1 ou 2, dans lequel les parois (210) du cadre (21) sont en appui contre les moyens de positionnement (204) lorsque le cadre (21) est dans la position de rangement.

4. Dispositif de stockage selon l'une des revendications 1 à 3, dans lequel les moyens de positionnement (204) comprennent un élément d'assemblage adapté à coopérer par complémentarité de formes avec le cadre (21).

5. Dispositif de stockage selon l'une des revendications précédentes, dans lequel les moyens de positionnement (204) sont adaptés à immobiliser le cadre (21) lorsque ledit cadre (21) est dans la position de rangement.

6. Dispositif de stockage selon l'une des revendications 1 ou 2, dans lequel un jeu sépare les moyens de positionnement (204) d'au moins une paroi (210) du cadre (21) lorsque le cadre (21) est dans la position de rangement, ledit jeu étant configuré de sorte à ce que lesdites parois (210) butent contre lesdits moyens de positionnement (204) lors de l'extraction et l'introduction du fil-guide (1) dans le cadre (21).

7. Dispositif de stockage selon l'une des revendications précédentes, dans lequel la partie ouverte de la gorge comprend des ponts (215) espacés le long de ladite partie ouverte et s'étendant d'un côté à un autre côté de ladite gorge (214).

8. Dispositif de stockage selon l'une des revendications précédentes, dans lequel la gorge (214) comprend une partie supérieure (214b) formée dans une portion supérieure (21b) du cadre (21) et une partie inférieure (214a) formée dans une portion inférieure (21a) du cadre (21), lesdites parties supérieure (214b) et inférieure (214a) de la gorge (214) étant semi-annulaires, ladite gorge (214) étant fermée sur la partie supérieure (214b) et débouchant sur l'ouverture (212) sur une ou plusieurs portions de la partie inférieure (214a), ledit orifice d'introduction (213) s'étendant dans la portion supérieure (214b) du cadre (21).

9. Dispositif de stockage selon l'une des revendications précédentes, dans lequel l'ouverture (212) est sensiblement circulaire.

10. Dispositif de stockage selon l'une des revendications précédentes, dans lequel les parois (210) du cadre (21) sont courbes.

11. Dispositif de stockage selon l'une des revendications précédentes, dans lequel le dispositif comporte un élément introducteur (22) communiquant avec le cadre (21) à travers l'orifice d'introduction (213).

12. Dispositif de stockage selon la revendication 11, dans lequel ledit élément introducteur (22) présente une forme sensiblement tronconique.

13. Dispositif de stockage selon la revendication 11, dans lequel ledit élément introducteur (22) comporte une portion tronconique (22a) et un bec d'introduction (22b) prolongeant ladite portion tronconique (22a) au niveau d'une extrémité évasée.

## Patentansprüche

1. Aufbewahrungsvorrichtung für Führungsdraht (1), umfassend einen Behälter (20) und mindestens einen abnehmbaren Rahmen (21), der geeignet ist, in dem Behälter (20) angeordnet zu werden, wobei der Behälter (20) dazu bestimmt ist, physiologische Serumlösung zu enthalten, wobei der Rahmen (21) zwei gegenüberliegende Wände (210) aufweist, die zwischen sich ein inneres Aufbewahrungsvolumen bilden und eine Einführungsöffnung (213) umfassen, die in dem inneren Volumen ausgebildet ist und sich von einem ersten Ende (210a) der Wände (210) in Richtung eines zweiten Endes (210b) der Wände (210) gegenüber dem ersten Ende (210a) erstreckt,
wobei der Rahmen (21) eine Öffnung (212) umfasst, die den Rahmen (21) von einer Wand (210) zur anderen durchdringt, und eine im Wesentlichen ringförmige Nut (214), die in dem inneren Volumen ausgebildet ist und mit der Einführungsöffnung (213) in Verbindung steht, wobei die Nut (214) über einen Teil ihres Umfangs, der als offener Teil bezeichnet wird, in die Öffnung (212) mündet und über den Rest ihres Umfangs von der Öffnung (212) getrennt ist, und der Behälter (20) Positionierungsmittel (204) umfasst, die dazu geeignet sind, den Rahmen (21) in eine Aufbewahrungsposition zu bringen, in der sich der offene Teil der Nut (214) wenigstens teilweise innerhalb des Behälters (20) befindet und die Einführungsöffnung (213) sich außerhalb des Behälters (20) erstreckt, wobei der Behälter (20) weiter Arettierungsmittel (203) umfasst, die dazu geeignet sind, die Translation des Rahmens (21) in Richtungen zum Einführen und Herausziehen des Führungsdrahts in den Rahmen (21) zu blockieren, wenn sich der Rahmen (21) in der Aufbewahrungsposition befindet.

2. Aufbewahrungsvorrichtung nach Anspruch 1, wobei die Positionierungsmittel (204) dazu geeignet sind, die Translation des Rahmens (21) gemäß einer seitlichen Richtung zu blockieren.

3. Aufbewahrungsvorrichtung nach einem der Ansprüche 1 oder 2, wobei die Wände (210) des Rahmens (21) an den Positionierungsmitteln (204) anliegen, wenn sich der Rahmen (21) in der Aufbewahrungsvorrichtung befindet.

4. Aufbewahrungsvorrichtung nach einem der Ansprüche 1 bis 3, wobei die Positionierungsmittel (204) ein Verbindungselement umfassen, das dazu geeignet ist, formschlüssig mit dem Rahmen (21) zusammenzuwirken.

5. Aufbewahrungsvorrichtung nach einem der vorhergehenden Ansprüche, wobei die Positionierungsmittel (204) dazu geeignet sind, den Rahmen (21) zu fixieren, wenn sich der Rahmen (21) in der Aufbewahrungsposition befindet.

6. Aufbewahrungsvorrichtung nach einem der Ansprüche 1 oder 2, wobei ein Spielraum die Positionierungsmittel (204) von mindestens einer Wand (210) des Rahmens (21) trennt, wenn sich der Rahmen (21) in der Aufbewahrungsposition befindet, wobei der Spielraum so konfiguriert ist, dass die Wände (210) beim Herausziehen und Einführen des Führungsdrahts (1) in den Rahmen (21) gegen die Positionierungsmittel (204) stoßen.

7. Aufbewahrungsvorrichtung nach einem der vorhergehenden Ansprüche, wobei der offene Teil der Nut Brücken (215) umfasst, die entlang des offenen Teils beabstandet sind und sich von einer Seite zu einer anderen Seite der Nut (214) erstrecken.

8. Aufbewahrungsvorrichtung nach einem der vorhergehenden Ansprüche, wobei die Nut (214) einen oberen Teil (214b), der in einem oberen Abschnitt (21b) des Rahmens (21) ausgebildet ist, und einen unteren Teil (214a), der in einem unteren Abschnitt (21a) des Rahmens (21) ausgebildet ist, umfasst, wobei der obere (214b) und der untere (214a) Teil der Nut (214) halbringförmig sind, wobei die Nut (214) am oberen Teil (214b) geschlossen ist und an der Öffnung (212) an einem oder mehreren Abschnitten des unteren Teils (214a) mündet, wobei sich die Einführungsöffnung (213) in den oberen Abschnitt (214b) des Rahmens (21) erstreckt.

9. Aufbewahrungsvorrichtung nach einem der vorhergehenden Ansprüche, wobei die Öffnung (212) im Wesentlichen kreisförmig ist.

10. Aufbewahrungsvorrichtung nach einem der vorhergehenden Ansprüche, wobei die Wände (210) des Rahmens (21) gekrümmt sind.

11. Aufbewahrungsvorrichtung nach einem der vorhergehenden Ansprüche, wobei die Vorrichtung ein Einführungselement (22) aufweist, das über die Einführungsöffnung (213) mit dem Rahmen (21) in Verbindung steht.

12. Aufbewahrungsvorrichtung nach Anspruch 11, wobei das Einführungselement (22) eine im Wesentlichen kegelstumpfförmige Form aufweist.

13. Aufbewahrungsvorrichtung nach Anspruch 11, wobei das Einführungselement (22) einen kegelstumpfförmigen Abschnitt (22a) und eine Einführungsnase (22b) aufweist, die den kegelstumpfförmigen Abschnitt (22a) an einem aufgeweiteten Ende verlängert.

## Claims

1. A storage device for a guide wire (1), including a tank (20) and at least one removable frame (21) adapted to be disposed in the tank (20), said tank (20) being intended to contain physiological serum, said frame (21) including two opposite walls (210) forming therebetween an inner storage volume and comprising an insertion orifice (213) formed in said inner volume and extending from a first end (210a) of said walls (210) in the direction of a second end (210b) of said walls (210) opposite to said first end (210a), said frame (21) comprising an opening (212) crossing the frame (21) from one wall (210) to the other, and a substantially annular groove (214) formed in said inner volume and communicating with said insertion orifice (213), said groove (214) leading into the opening (212) over a portion of its perimeter, called open portion, and being separated from the opening (212) over the rest of its perimeter, and said tank (20) including positioning means (204) adapted to place the frame (21) in a stowage position in which the open portion of the groove (214) is located at least partially inside the tank (20) and the insertion orifice (213) extends outside the tank (20), said tank (20) further including stop means (203) adapted to block the translation of the frame (21) in directions of insertion and extraction of the guide wire into and from the frame (21) when said frame (21) is in the stowage position.

2. The storage device according to claim 1, wherein the positioning means (204) are adapted to block the translation of the frame (21) according to a lateral direction.

3. The storage device according to one of claims 1 or 2, wherein the walls (210) of the frame (21) bear against the positioning means (204) when the frame (21) is in the stowage position.

4. The storage device according to one of claims 1 to 3, wherein the positioning means (204) comprise an assembly element adapted to cooperate with the frame (21) in a formfitting manner.

5. The storage device according to one of the preceding claims, wherein the positioning means (204) are adapted to immobilise the frame (21) when said frame (21) is in the stowage position.

6. The storage device according to one of claims 1 or 2, wherein a clearance separates the positioning means (204) from at least one wall (210) of the frame (21) when the frame (21) is in the stowage position, said clearance being configured so that said walls (210) abut against said positioning means (204) during the extraction and insertion of the guide wire (1) from and into the frame (21).

7. The storage device according to one of the preceding claims, wherein the open portion of the groove comprises bridges (215) spaced apart along said open portion and extending from one side to another side of said groove (214).

8. The storage device according to one of the preceding claims, wherein the groove (214) comprises an upper portion (214b) formed in an upper portion (21b) of the frame (21) and a lower portion (214a) formed in a lower portion (21a) of the frame (21), said upper (214b) and lower (214a) portions of the groove (214) being semiannular, said groove (214) being closed on the upper portion (214b) and leading into the opening (212) on one or more portions of the lower portion (214a), said insertion orifice (213) extending in the upper portion (214b) of the frame (21).

9. The storage device according to one of the preceding claims, wherein the opening (212) is substantially circular.

10. The storage device according to one of the preceding claims, wherein the walls (210) of the frame (21) are curved.

11. The storage device according to one of the preceding claims, wherein the device includes an inserter element (22) communicating with the frame (21) through the insertion orifice (213).

12. The storage device according to claim 11, wherein said inserter element (22) has a substantially frustoconical shape.

13. The storage device according to claim 11, wherein said inserter element (22) includes a frustoconical portion (22a) and an insertion spout (22b) extending said frustoconical portion (22a) at a flared end.
